# EUROPEAN PATENT SPECIFICATION

(11) **EP 1 530 455 B1**
(45) Date of publication and mention of the grant of the patent: **18.03.2009**
(21) Application number: 03766101.4
(22) Date of filing: 30.07.2003
(51) Int. Cl.: A61K 9/08, A61K 31/495

(54) **A PHARMACEUTICAL FORMULATION AND ITS USE IN THE TREATMENT OF INNER EAR DISEASES**
ARZNEIZUBEREITUNG UND DEREN ANWENDUNG ZUR BEHANDLUNG VON ERKRANKUNGEN DES INNEREN OHRES
FORMULATION PHARMACEUTIQUE ET SON UTILISATION DANS LE TRAITEMENT DES MALADIES DE L'OREILLE INTERNE

(30) Priority: 31.07.2002 CH 135702
(43) Date of publication of application: 18.05.2005
(73) Proprietor: PHAFAG AKTIENGESELLSCHAFT, 9496 Schaanwald (LI)
(72) Inventor: BIEBERSCHULTE, Werner, LI-9492 Eschen (LI); EHRENBERGER, Klaus, A-1090 Wien (AT)
(74) Representative: Hasler, Erich
(86) International application number: PCT/CH2003/000523
(87) International publication number: WO 2004/012705

(56) References cited:
- EP-A- 0 032 564
- EP-A- 0 542 689
- WO-A-92/00729
- WO-A-92/04011
- WO-A-99/66931
- DATABASE WPI Section Ch, Week 198319 Derwent Publications Ltd., London, GB; Class A96, AN 1983-45789K XP002235212 & JP 58 057317 A (MITSUBISHI CHEM IND LTD) , 5 April 1983 (1983-04-05)

## Description

The invention concerns a pharmaceutical formulation as well as the use of this formulation for the treatment especially of inner ear diseases.

Quinoxaline-2-one derivatives have been known since the 60's or early 70's as active pharmaceutical compounds. An important representative of the quinoxaline-2-one derivatives is Caroverin (= non-proprietary name) whose correct chemical name is 1-diethylaminoethyl-3-(p-methoxybenzyl)-1,2-dihydro-quinoxaline-2-one.

EP-A-0 542 689 describes the use of quinoxaline-2-one derivatives for the production of effective pharmaceutical, neuro-protective compounds for the treatment of neuro-toxicities and functional disturbances of the central nervous system. According to EP-A-0 542 689, Caroverin is especially useful in the treatment of cochlear post-synaptic tinnitus. The previously described, pharmaceutically effective quinoxaline-2-one derivatives should be able to be administered orally, rectally or parenterally. In a clinical trial on the treatment of cochlear post-synaptic tinnitus, the patient was administered between 70 and 160 mg of Caroverin dissolved in 100 ml of physiological saline, intravenously.

WO 99/66931 proposes the use of quinoxaline derivatives for the treatment of diseases caused by the presence of free-radicals of cell-oxygen metabolism, for stimulation of nerve cell synthesis and for antagonizing glutamate receptors, etc. According to WO 99/66931, the quinoxaline derivates referred to can be administered by any known method, especially oral, trans-dermal, topical and parenteral, although the intra-venous route is preferred. The topical administration of Caroverin in the form of an ointment has been described for the alleviation of sun injuries as well as of skin inflammation caused by oxidation. For these indications, it is a matter of treating superficially and locally with the active medication, which need not necessarily penetrate the blood circulation system for the activity to be effective. In the example of its use mentioned in WO 99/66931, Caroverin was administered once or more frequently to the patient intravenously in relatively high doses. The dosage used was between 160 mg to 1000 mg per day. For many indications, the degree of effectiveness of Caroverin was strongly dependent on the dosage given, and was only satisfactory at high doses. For example, to achieve a neuro-regenerative effect, it was necessary to administer higher doses of Caroverin.

In view of the very high doses required, the specialists did not previously consider the administration of Caroverin by a trans-dermal route, since with trans-dermal administration, it is well known that only relatively small active amounts may be introduced into the blood stream. Trials undertaken by the inventor have shown also that Caroverin only has a limited permeability through the skin.

In addition, new trials by the inventor have shown that Caroverin is not indicated for the treatment of all forms of tinnitus. Apart from cochlear post-synaptic tinnitus, which, according to EP 0 542 689, is treatable by infusion of Caroverin, it has been observed that a muscular tinnitus (also known as myognathic tinnitus) is not responsive to the intravenous administration of Caroverin. Muscular or myognathic tinnitus is identified by imparting an electrical charge to the chewing musculature, whereby the intensity and frequency of the symptoms of tinnitus can be altered. Myognathic tinnitus can substantially be caused to disappear when the tendon bundles of the middle ear musculature that operates the chain of auditory ossicles are surgically sectioned (tenotomy). The precise mechanism involved in this effect is still unclear.

Morbus Menière, named after the French doctor of the same name, is a relatively rare disease, the symptoms of which are turning-giddiness, ringing in the ears and noise-sensitive hearing deficiency. The disease appears from time to time, and is usually accompanied by severe nausea and vomiting. After some time, a pan-cochlear loss of hearing develops. At the present time, the recommended medical therapies for Morbus Ménière are highly unsatisfactory.

It is, therefore, an object of this invention, to make available an improved pharmaceutical preparation or formulation for the treatment of inner ear diseases. An object of this invention is to provide an improved formulation that can also be administered by the patient himself. Yet another object is to make available a medicine and/or a pharmaceutical formulation for the treatment also of the so-called muscular or myognathic tinnitus, Morbus Ménière, labyrinthine vertigo and impairment of hearing, especially such associated with speech comprehension deficiency.

According to the present invention, the object is achieved by the use of a a quinoxalin-2-one derivative of the formula 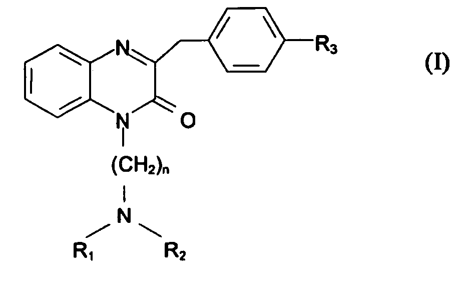
in which R₁ and R₂, independently of one another, are hydrogen, methyl-, ethyl-, propyl- or butyl-, or R1 and R2 together form a cyclo-alkyl compound.
R3 is methoxy, ethoxy, hydroxy, hydrogen, C1-C4 alkyl or halogen;
and n = 1, 2 or 3, or a pharmaceutically compatible salt of the aforesaid derivatives; and, further containing a permeability accelerator or carrier in respect of the afore-mentioned quinoxalin-2-one derivatives; as well as, if required, a pharmaceutically compatible solvent, for the production of a liquid pharmaceutical formulation, also in the form of liposomes or a nano-emulsion, for trans-tympanic administration.

The inventor was surprised to discover that, with the help of a suitable permeability accelerator, or activator, and carrier system, respectively, a formulation could be identified that could provide trans-tympanic administration, and that was already highly effective with low dosages. This result was completely unexpected since it had been necessary to administer relatively high doses of quinoxalin-2-one derivatives intravenously in the case of the abovementioned indications. Therefore, it could not have been expected that the quinoxalin-2-one derivatives together with a suitable permeability accelerator or carrier system were already effective at much lower dosages when the novel formulation was applied through the tympanum.

Especially effective are the quinoxalin-2-one derivatives in which the R1 and R2 substituents are an ethyl group, where n = 2, and in which R3 is a methoxy group, so that the molecule is 1-diethylaminoethyl-3-(p-methoxybenzyl)-1,2-dihydro-quinoxalin-2-one (non-proprietary name: Caroverin) or a pharmaceutically compatible salt thereof. Furthermore, quinoxalin-2-one derivatives are also effective in which the R1 and R2 subsituents are an ethyl group where n = 2, and R3 is a hydroxy group, so that the molecule is 1-diethylaminoethyl-3-(p-hydroxybenzyl)-1,2-dihydro-quinoxalin-2-one or a pharmaceutically compatible salt thereof.

Advantageously, the permeability accelerator used in the formulation is one of the following compounds: dimethyl sulphoxide, mono-glyceride, ethyl- or methyl-palmitic acid ester, fatty acids, fatty acid esters, fatty acid alcohols, substituted dialkyl fatty acids having 8 to 14 carbon atoms, N-methyl pyrrolidone, N-methyl-2-pyrrolidone oleic acid, propylene glycol, diethylene glycol, the mono-alkzyl ether or carboxy-methyl ether of polyethylene glycol, propylene glycolfatty acid ester, lauryl acetate, N,N-dialkyl lauramide, histamine, a dialkyl lauramide/dimethyl formamide mixture, dimethyl acetamide, N,N-diethyl-m-toluamide, ethylene glycol monomethyl ether, isopropyl myristate, isopropyl palmitate, propylene glycol and oleic acid or oleyl alcohol, 2-pyrrolidone and dimethyl formamide, lauric acid, linoleic acid, lauryl acetate, sodium oleate, glycerine mono-oleate, urea, 1-bisabolol. Also possible is the use of a combination of 2 or more of the aforementioned permeability accelerators.

In a preferred formulation, the permeability accelerator used is at least dimethyl sulphoxide or propylene glycol. It has been shown that dimethyl sulphoxide is a good solvent and, surprisingly, a good permeability accelerator for quinoxalin-2-one derivatives, especially for Caroverin. Furthermore, dimethyl sulphoxide can be added in fairly high concentrations. The content by weight of dimethyl sulphoxide in the formulation preferably comprises between 5 and 50%, but can also be higher. Advantageously, the formulation contains, together with dimethyl sulphoxide, at least another second permeability accelerator. This can be a member of the above-mentioned group of permeability accelerators. By the addition of at least one further permeability accelerator, the content by weight of dimethyl sulphoxide can be correspondingly reduced. In this way, the risk is reduced that the administration of the formulation causes skin irritation.

Preferably, the second permeability accelerator is a glycol compound such, for example, as an ethylene- and/or propylene glycol compound.

Advantageously, the ratio by weight of quinoxalin-2-one derivative to the permeability accelerator is between 1:2 and 1:500, preferably between 1:20 and 1:100. As the solvent, for example, glycerine or another physiologically compatible compound, such as water, can be used.

An advantageous formulation uses a nanoemulsion or liposomes, which contain the said quinoxalon-2-one compound according to Formula (I), as the permeation accelerator or carrier. Expediently, the nanoemulsion or the liposomes contain a membrane-forming molecule and a coemulsifier besides the said quinoxalon-2-one derivatives.

Substances which make it possible to form two-layer systems (so-called "bilayers") are preferably used as the membrane-forming molecule. A phospholipid is preferably used as the membrane-forming molecule. It may be a hydrogenated or partially hydrogenated phospholipid. A naturally or artificially produced lecithin is preferably used. The latter may, for example, be obtained from soy beans or hen's eggs. Examples of phospholipids are phosphatidylcholine, phosphatidylethanolamine, phosphatidylglycerol, phosphatidylinositol, phosphatidylserine and sphingomyelin. The acyl chain may be either saturated or unsaturated, and may have from 12 to 22, preferably from 14 to 18 C atoms. Other liposome-forming membrane lipids such as glycolipids, ceramides, gangliosides and cerebrosides may be used instead of, or partially instead of, phospholipids.

The lipids may be derived from natural plant, animal or microbiological sources, synthetically produced or partially synthetically produced, inclusive of monoacyl phospholipids derived from polyethylene glycol (PEG), for example pegylated monoacyl phosphatidylethanolamine.

According to a particularly preferred embodiment, a phospholipid of the formula 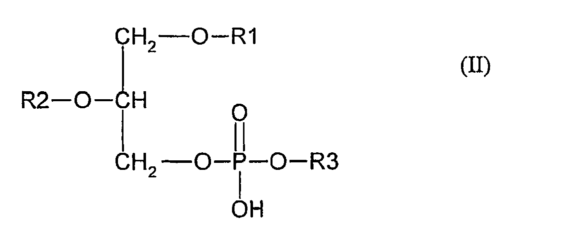 in which
R1 denotes C₁₀-C₂₀ acyl;
R2 denotes hydrogen or C₁₀-C₂₀ acyl
R3 denotes hydrogen, 2-trimethylamino-1-ethyl, 2-amino-1-ethyl, unsubstituted C₁-C₅ alkyl or C₁-C₅ alkyl substituted with one or more carboxyl, hydroxy or amino groups; the inositol or glyceryl group, or salts of these compounds,
is used as the membrane-forming molecule.

The C₁₀-C₂₀ acyl is preferably a straight-chained C₁₀-C₂₀ alkanoyl having an even number of C atoms or straight-chained C₁₀₋C₂₀ alkanoyl having one or more double bonds and an even number of C atoms. Straight-chained C₁₀-C₂₀ alkanoyls having an even number of C atoms are, for example, n-dodecanoyl, n-tetradecanoyl, n-hexadecanoyl or n-octadecanoyl. Straight-chained C₁₀-C₂₀ alkanoyls having a double bond and an even number of C atoms are, for example, 6-cis- or 6-trans-, 9-cis- or 9-trans-dodecenoyl, - tetradecenoyl, -hexadecenoyl, -octadecenoyl or -icosenoyl, in particular 9-cis-octadecenoyl (oleoyl), and 9,12-cis-octadecadienoyl or 9,12,15-cis-octadecatrienoyl.

A phospholipid of Formula (II) in which R3 denotes 2-trimethylamino-1-ethyl is referred to by the trivial name lecithin, and a phospholipid of Formula (II) in which R3 denotes 2-amino-1-ethyl is referred to by the trivial name cephalin. For example, naturally occurring cephalin or lecithin having different or identical acyl groups, or mixtures thereof, are suitable.

The membrane-forming component is preferably used in a concentration of about 0.1 to 30% by weight, expressed in terms of the total weight of membrane-forming component, emulsifier and active substance.

One of the following coemulsifiers, or an emulsifier mixture of two or more of the coemulsifiers listed below, may be used as the emulsifier:

Alkali metal, ammonium and aminium salts of fatty acids, for example lithium, sodium, potassium, ammonium, triethylamine, ethanolamine, diethanolamine or triethanolamine salts. Sodium, potassium or ammonium (NR₁R₂R₃) salts are preferred, R₁, R₂ and R₃ independently of one another denoting hydrogen, C₁-C₄ alkyl or C₁-C₄ hydroxyalkyl. Alkyl sulfates such as, for example, sodium dodecyl sulphate.
Salts of bile acid, for example sodium cholate, sodium glycocholate and sodium taurocholate;
partial fatty acid esters of sorbitan, for example sorbitan monolaurate;
sugar esters, for example sucrose monolaurate;
fatty acid partial glycerides, for example lauric acid monoglyceride;
polyglycerol esters of fatty acids;
propylene glycol esters of fatty acids;
lactic acid esters of fatty acids, for example sodium stearoyl-lactyl-2-lactate;
proteins, for example casein.

Emulsifiers of the polyoxyethylene type are more particularly preferred. Examples of such emulsifiers are:
- polyethoxylated sorbitan fatty acid esters, for example polysorbate 80;
- polyethoxylated vitamin E derivates, for example vitamin E polyethylene glycol 1000 succinate;
- polyethoxylated fatty acid partial glycerides, for example diethylene glycol monostearate;
- polyethoxylated carbohydrates;
- block polymers of ethylene oxide and propylene oxide, for example poloxamer 188.

The emulsifier is advantageously present in the formulation at a concentration of about 1 to about 50% by weight, expressed in terms of the total weight of the membrane-forming component, the emulsifier and the active substance. The quinoxalin-2-one compound is present in the formulation at a concentration of about 0.1 to 70% by weight, expressed in terms of the total weight of the components (membrane-forming component, emulsifier and quinoxalin-2-one compound).

The subject of the present invention also comprises the use of a quinoxalin-2-one derivative of the formula: 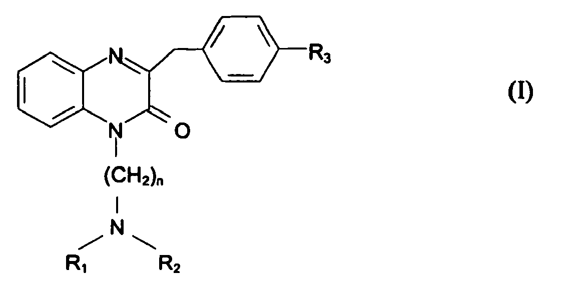
in which R1 and R2, independently of one another, are hydrogen, methyl-, ethyl-, propyl- or butyl-, or R1 and R2 together form a dyclo-alkyl compound;
R3 is the methoxy, ethoxy, hydroxy, hydrogen, a C1-C4 alkyl or halogenatom, and n = 1, 2 or 3;
or a pharmaceutically compatible salt of the afore-mentioned quinoxalin-2-one compound; and
an effective amount of a compound that is a permeability accelerator, also in the form of a suitable carrier like a nanoemulsion or liposomes, in respect of the above-mentioned quinoxalin-2-one derivatives for the manufacture of a pharmaceutical formulation that can be administered through the tympanum for the treatment of inner-ear diseases. Preferably, Caroverin or a salt compound of Caroverin is used in the formulation. Salt compounds have the advantage that they are more readily soluble in divers solvents.

The formulation according to the invention is preferably administered in a liquid form. Aqueous formulations as well as non aqueous ones can be used. Advantageously the viscosity of the formulation is between 5000 and 25000 mPas (milli-Pascal per second), preferably between 15000 and 20000 mPas, so that a longer period of administration of the active ingredient into the inner ear is achieved. The part by weight of quinoxalin-2-one derivative used is preferably between about 0.5% and 12%.

The treatment of a patient takes place as follows: An absorbable material such, for example, as a wick of about 2 mm diameter and 2 to 3 cm in length is soaked with the inventive formulation. The soaked wick is then inserted into the ear so that it lies against the tympanic membrane. Depending on the concentration of the solution used, the therapy lasts between 3 and 24 hours. Depending on the condition of the disease, the above described treatment is continued by the additional administration of a specific number of drops of the active formulation, such, for example, as 3 - 4 drops every second day.

In the case where the formulation is a nanoemulsion, the formulation can also be applied directly onto tympanum.

Alternatively, the formulation can also be administered intra-trans-tympanically. For this form of administration of active substances, a canula or drainage tubule that reaches into the middle ear, is used and the active substances are administered via the drainage tubule.

### Examples of the Formulation

Three examples of the non-aqueous formulations are as follows:

| Example 1 | Example 2 |
|---|---|
| 0.5% of Caroverin | 0.5% of Caroverin |
| 20% of dimethyl sulphoxide | 20% of dimethyl sulphoxide |
| 30% of propylene glycol | 30% of propylene glycol |
| 50% of glycerine | 50% of gel |

| Example 3 | Example 4 |
|---|---|
| 10% of Caroverin hydrochloride | 1 % of Caroverin |
| 20% of dimethyl sulphoxide | 3% of acetone |
| 30% of propylene glycol | 96% of propylene glycol |
| 40% of glycerine | |

The viscosity of the different trial solutions (formulations) is, preferably, between 5000 and 25000 mPas, more preferably between 15000 - 20000mPas.

The following example is of an aqueous formulation with a thickening agent:

### Example 5

1% of Caroverin hydrochloride
20% of dimethyl sulphoxide
30% of propylene glycol
48% of water
1% of thickening agent (PVM/MA decadiene cross-polymer).

The viscosity of the solution is preferably increased by the addition of a thickening agent, so that, during the drop-wise administration of the solution into the outer auditory meatus the solution stays as long as possible on the tympanic membrane without running out. Because of the good adhesion of the formulation on the tympanic membrane, a long lasting administration of the active ingredient into the inner ear is ensured.

### Preparation of a nanoemulsion / liposomes

In order to produce the formulation, the emulsifier and the quinoxalin-2-one compound are mixed to form a homogenous liquid phase, optionally while heating. The phospholipid is dissolved in this phase, optionally with the aid of a solubility promoter, for example ethanol. This results in a homogenous solution. It may then be diluted with water to the intended concentration of active substance.

## Claims

1. The use of a quinoxalin-2-one compound of the formula 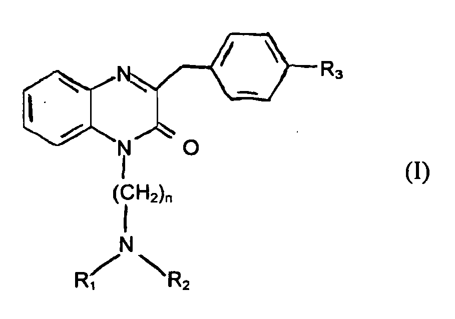 according to which
R1 and R2, independently of one another, are hydrogen, methyl-, ethyl-, propyl- or butyl- , or R1 and R2 together form a cyclo-alkyl compound;
R3 is methoxy , ethoxy, hydroxy, hydrogen, C1-C4 alkyl or halogen; and n = 1,2 or 3,
or a pharmaceutically compatible salt of the afore-mentioned quinoxalin-2-one compound, together with a permeability accelerator or carrier in respect of the quinoxalin-2-one compound, for the production of a pharmaceutical formulation in the form of a liquid or liposomes or a nano-emulsion for trans-tympanic administration.

2. The use as claimed in Claim 1, according to which R1 and R2 are ethyl groups, n = 2 and R3 is a methoxy group, so that the molecule is 1-diethyl-aminoethyl-3-(p-methoxybenzyl)-1,2-dihydro quinoxalin-2-one or a pharmaceutically compatible salt thereof.

3. The use as claimed in Claim 1, according to which R1 and R2 are ethyl groups, n = 2 and R3 is a hydroxy group, so that the molecule is 1-diethyl-aminoethyl-3-(p-hydroxybenzyl)-1,2-dihydro quinoxalin-2-one or a pharmaceutically compatible salt thereof.

4. The use as claimed in any one of the Claims 1 to 3, according to which the permeability accelerator at least contains dimethyl sulphoxide or propylene glycol.

5. The use as claimed in Claim 4, according to which the part by weight of dimethyl suphoxide used in the formulation is between 5 and 50%.

6. The use as claimed in any one of the Claims 1 to 5, according to which at least one further second permeability accelerator is contained in combination with dimethyl sulphoxide.

7. The use as claimed in Claim 6, according to which the second permeability accelerator used is a glycol compound.

8. The use as claimed in Claim 6 or 7, according to which the second permeability accelerator used is ethylene- and/or propylene glycol.

9. The use as claimed in any one of the Claims 1 to 8, according to which the ratio by weight of quinoxalin-2-one to the permeability accelerator is between 1:2 and 1:500, preferably between 1:20 and 1:100.

10. The use as claimed in any one of the Claims 1 to 9, according to which additionally a solvent is used, which is glycerine and/or water.

11. The use as claimed in any one of the Claims 1, according to which a nanoemulsion or liposomes, which contain the said quinoxalon-2-one compound according to Formula (I), are used as a permeation accelerator or carrier.

12. The use as claimed in Claim 11, according to which the nanoemulsion or the liposomes contain the following compounds besides the said quinoxalon-2-one compound:
- a membrane-forming molecule and
- a coemulsifier.

13. The use as claimed in Claim 12, according to which the membrane-forming molecule is a substance which make it possible to form two-layer system.

14. The use as claimed in Claim 12 or 13, according to which the membrane-forming molecule is a hydrogenated or partially hydrogenated phospholipids.

15. The use as claimed in Claim 14, according to which the phospholipids is one phosphatidylcholine, phosphatidylethanolamine, phosphatidylglycerol, phosphatidylinositol, phosphatidylserine and sphingomyelin wherein the acyl chain may be either saturated or unsaturated, and may have from 12 to 22, preferably from 14 to 18 C atoms.

16. The use as claimed in Claim 14 or 15, according to which the phospholipid has the formula 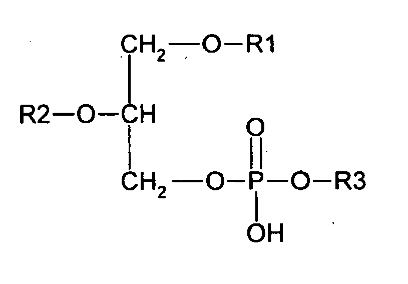 in which
R1 denotes C₁₀-C₂₀ acyl;
R2denotes hydrogen or C₁₀-C₂₀ acyl
R3denotes hydrogen, 2-trimethylamino-1-ethyl, 2-amino-1-ethyl, unsubstituted C₁-C₅ alkyl or C₁-C₅ alkyl substituted with one or more carboxyl, hydroxy or amino groups; the inositol or glyceryl group, or salts of these compounds,
is used as the membrane-forming molecule

17. The use as claimed in any of Claims 12 to 16,according to which the membrane-forming component is used in a concentration of about 0.1 to 30% by weight, expressed in terms of the total weight of membrane-forming component, emulsifier and active substance.

18. The use as claimed in Claim 12, according to which the following coemulsifiers, or an emulsifier mixture of two or more of the coemulsifiers listed below, is used:
Alkali metal, ammonium and aminium salts of fatty acids, for example lithium, sodium, potassium, ammonium, triethylamine, ethanolamine, diethanolamine or triethanolamine salts. Sodium, potassium or ammonium (NR₁R₂R₃) salts are preferred, R₁, R₂ and R₃ independently of one another denoting hydrogen, C₁-C₄ alkyl or C₁-C₄ hydroxyalkyl.
Alkyl sulfates such as, for example, sodium dodecyl sulphate.
Salts of bile acid, for example sodium cholate, sodium glycocholate and sodium taurocholate;
partial fatty acid esters of sorbitan, for example sorbitan monolaurate;
sugar esters, for example sucrose monolaurate;
fatty acid partial glycerides, for example lauric acid monoglyceride;
polyglycerol esters of fatty acids;
propylene glycol esters of fatty acids;
lactic acid esters of fatty acids, for example sodium stearoyl-lactyl-2-lactate;
proteins, for example casein.

19. The use as claimed in Claim 18, according to which the emulsifiers of the polyoxyethylene type are:
- polyethoxylated sorbitan fatty acid esters, for example polysorbate 80;
- polyethoxylated vitamin E derivates, for example vitamin E polyethylene glycol 1000 succinate;
- polyethoxylated fatty acid partial glycerides, for example diethylene glycol monostearate;
- polyethoxylated carbohydrates;
- block polymers of ethylene oxide and propylene oxide, for example poloxamer **188.**

20. The use as claimed in any of Claims 12 to 19,according to which the emulsifier is present in the formulation at a concentration of about 1 to about 50% by weight, expressed in terms of the total weight of the membrane-forming component, the emulsifier and the active substance.

21. The use as claimed in any of Claims 12 to 20,according to which the quinoxalin-2-one compound is present in the formulation at a concentration of about 0.1 to 70% by weight, expressed in terms of the total weight of the components (membrane-forming component, emulsifier and quinoxalin-2-one compound).

22. The use as claimed in any one of the Claims 1 to 12, according to which the formulation is liquid and the part by weight of the quinoxalin-2-one compound is between 0.5% and 12%.

23. The use as claimed in any one of the Claims 1 to 12, according to which the formulation is used either as a non-aqueous or as an aqueous formulation.

24. The use as claimed in any one of the Claims 1 to 12, according to which acetone and propylene glycol are used as permeation accelerators.

25. The use as claimed in any one of the Claims 1 to 24, according to which it is used for the treatment of inner ear diseases.

26. The use as claimed in any one of the Claims 1 to 24, according to which it is used for the treatment of muscular or myognathic tinnitus.

27. The use as claimed in any one of the Claims 1 to 24, according to which it is used for the treatment of Morbus Ménière.

28. The use as claimed in any one of the Claims 1 to 24, according to which it is used for the treatment of speech-discrimination deficiency, especially in combination with hearing deficiency.

29. The use as claimed in any one of the Claims 1 to 24, according to which it is used for the treatment of labyrinthine vertigo.

## Patentansprüche

1. Verwendung einer Chinoxalin-2-on Verbindung der Formel 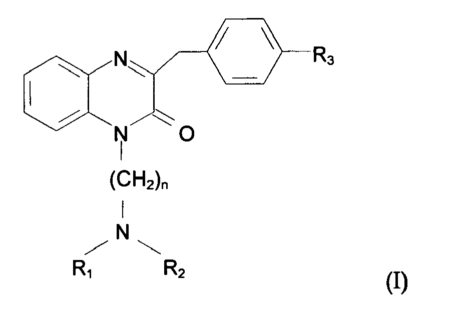
worin R1 und R2 unabhängig voneinander Wasserstoff, Methyl-, Ethyl-, Propyl-, Butyl-, sind oder R1 und R2 zusammen eine Cycloalkylverbindung bilden;
R3 ist Methoxy, Ethoxy, Hydroxy, Wasserstoff, C1-C4 Alkyl, Halogen; und
n=1,2 oder 3,
oder eine pharmazeutisch verträglichen Salz der erwähnten Chinoxalin-2-on Verbindung, zusammen mit einem Permeationsbeschleuniger oder Carrier bezüglich der erwähnten Chinoxalin-2-on Verbindung zur Herstellung einer pharmazeutischen Formulierung in der Form einer Flüssigkeit, von Liposomen, oder einer Nano-Emulsion zur transtympanalen Verabreichung.

2. Verwendung nach Anspruch 1, worin R1 und R2 eine Ethyl-Gruppe; n=2 und R3 eine Methoxy-Gruppe sind, sodass das Molekül 1-Diethylaminoethyl-3-(p-methoxybenzyl)-1,2-dihydro-Chinoxalin-2-on oder ein pharmazeutisch verträgliches Salz ist.

3. Verwendung nach Anspruch 1, worin R1 und R2 eine Ethyl-Gruppe, n=2 und R3 eine Hydroxy-Gruppe sind, sodass das Molekül 1-Diethylaminoethyl-3-(p-hydroxybenzyl)-1,2-dihydro-Chinoxalin-2-on oder ein pharmazeutisch verträgliches Salz davon ist.

4. Verwendung nach einem der Ansprüche 1 bis 3, **dadurch gekennzeichnet, dass** der Permeationsbeschleuniger wenigstens Dimethylsulfoxid oder Propylenglykol enthält.

5. Verwendung nach Anspruch 4, **dadurch gekennzeichnet, dass** der gewichtsmässige Anteil von Dimethylsulfoxid in der Formulierung zwischen 5 und 50% liegt.

6. Verwendung nach einem der Ansprüche 1 bis 5, **dadurch gekennzeichnet, dass** in Kombination mit Dimethylsulfoxid wenigstens ein weiterer zweiter Permeationsbeschleuniger enhalten ist.

7. Verwendung nach Anspruch 6, **dadurch gekennzeichnet, dass** der zweite Permeationsbeschleuniger eine Glykolverbindung ist.

8. Verwendung nach einem der Ansprüche 6 oder 7, **dadurch gekennzeichnet, dass** der zweite Permeationsbeschleuniger Ethylen- und/ oder Propylenglykol ist.

9. Verwendung nach einem der Ansprüche 1 bis 8, **dadurch gekennzeichnet, dass** das gewichtsmässige Verhältnis von Chinoxalin-2-on-Derivat und Permeationsbeschleuniger zwischen 1:2 und 1:500, vorzugsweise zwischen 1:20 und 1:100 liegt.

10. Verwendung nach einem der Ansprüche 1 bis 9, **dadurch gekennzeichnet, dass** als Lösungsmittel Glyzerin und/oder Wasser eingesetzt ist.

11. Verwendung nach einem der Ansprüche 1 bis 10, **dadurch gekennzeichnet, dass** eine Nano-Emulsion oder Liposomen, welche die genannte Chinoxalin-2-on Verbindung gemäss Formel (I) enthalten, als Permeationsbeschleuniger oder Carrier verwendet werden.

12. Verwendung nach Anspruch 11, **dadurch gekennzeichnet, dass** die Nano-Emulsion oder die Liposomen die folgenden Verbindungen neben der genannten Chinoxalin-2-on Verbindung enthalten:
- ein membranbildendes Molekül und
- einen Koemulgator.

13. Verwendung nach Anspruch 12, **dadurch gekennzeichnet, dass** das membranbildende Molekül eine Substanz ist, welche die Bildung eines Zwei-Lagen-Systems ermöglicht.

14. Verwendung nach einem der Ansprüche der Ansprüche 12 oder 13, **dadurch gekennzeichnet, dass** das membranbildende Molekül ein hydriertes oder teilweise hydriertes Phospholipid ist.

15. Verwendung nach Anspruch 14. **dadurch gekennzeichnet, dass** das Phospholipid ein Phosphatidylcholin, ein Phosphatidylathanolamin, ein Phosphatidylglycerol, ein Phosphatidylinositol, ein Phosphatidylserin, und ein Sphingomyelin ist, wobei die Acyl-Ketten gesättigt oder ungesättigt sein können und 12 bis 22, bevorzugt 14 bis 18 C-Atome aufweisen.

16. Verwendung nach einem der Ansprüche 14 bis 15, **dadurch gekennzeichnet, dass** das Phospholipid die Formel 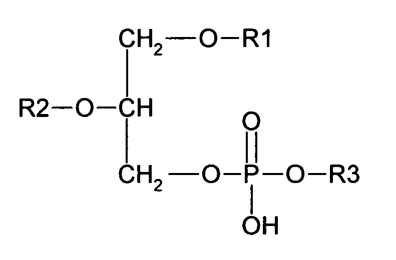 besitzt, wobei
R1 eine C₁₀-C₂₀ Acyl-Gruppe bezeichnet
R2 Wasserstoff oder eine C₁₀-C₂₀ Acyl-Gruppe bezeichnet
R3 Wasserstoff, 2-Trimethylamino-1-Ethyl, 2-Amino-1-Ethyl, eine unsubstituierte C₁-C₅ Alkyl-Gruppe oder eine mit einer oder mehreren Carboxyl-, Hydroxy- oder AminoGruppen substituierte C₁-C₅ Alkyl-Gruppe bezeichnet;
die Inositol- oder Glycerol-Gruppe, oder Salze dieser Verbindungen werden als das membranbildende Molekül verwendet.

17. Verwendung nach einem der Ansprüche 12 bis 16, **dadurch gekennzeichnet, dass** die membranbildende Komponente in einer Konzentration von 0.1 bis 30 Gewichts% verwendet wird, bezogen auf das Gesamtgewicht der membranbildenden Komponente, des Emulgators und der aktiven Substanz.

18. Verwendung nach Anspruch 12, **dadurch gekennzeichnet, dass** die folgenden Koemulgatoren, oder eine Emulgatormischung von zwei oder mehreren untenstehenden Koemulgatoren, verwendet wird:
Alkalimetalle, Ammonium- und Amminsalze von Fettsäuren, z.B. Lithium-, Natrium-, Kalium-, Ammonium-, Triethylamin-, Ethanolamin-, Diethanolamin- oder Triethanolaminsalze. Natrium-, Kalium- oder Ammoniumsalze (NR₁R₂R₃) sind bevorzugt, R1, R2 und R3, bezeichnend Wasserstoff, C₁-C₄ Alkyl- oder C₁-C₄ Hydroxyalkylgruppen, sind gegenseitig unabhängig.
Alkylsulfate wie z.B. Natriumdodecylsulfat.
Salze der Gallensäure, z.B. Natriumcholat, Natriumglykocholat und Natriumtaurocholat;
partielle Fettsäureester von Sorbitan, z.B. Sorbitanmonolaurat;
Zuckerester, z.B. Saccharosemonolaurat;
Teilglyceride von Fettsäuren, z.B. Laurinsäuremonoglycerid;
Polyglyzerinester von Fettsäuren;
Propylenglykolester von Fettsäuren;
Milchsäurester von Fettsäuren, z.B. Natriumstearyllaktyl-2-laktat;
Proteine, z.B. Kasein.

19. Verwendung nach Anspruch 18, **dadurch gekennzeichnet, dass** die Emulgatoren vom Polyoxyethylentyp sind:
- polyethoxylierte Sorbitanfettsäureester, z. B. Polysorbat 80;
- polyethoxylierte Vitamin E Derivate, z. B. Vitamin E Polyethylenglykol 1000 Succinat;
- Teilglyceride von polyethoxylierten Fettsäuren, z. B. Diethylenglykolmonostearat;
- polyethoxylierte Kohlenhydrate
- Blockpolymere aus Ethylenoxid und Propylenoxid, z. B. Poloxamer.

20. Verwendung nach einem der Ansprüche 12 bis 19, **dadurch gekennzeichnet, dass** der Emulgator in der Formulierung bei einer Konzentration von 1 bis 50 Gewichts% anwesend ist, bezogen auf das Gesamtgewicht der membranbildenden Komponente, des Emulgators und der aktiven Substanz.

21. Verwendung nach einem der Ansprüche 12 bis 20, **dadurch gekennzeichnet, dass** die Quinoxalin-2-on Verbindung in der Formulierung bei einer Konzentration von 0.1 bis 70 Gewichts% verwendet wird, bezogen auf das Gesamtgewicht der Komponenten (membranbildenden Komponente, Emulgators und Quinoxalin-2-on Verbindung).

22. Verwendung nach einem der Ansprüche 1 bis 12, **dadurch gekennzeichnet, dass** die Formulierung flüssig ist und der gewichtsmässige Anteil der Chinoxalin-2-on Verbindung zwischen 0.5% und 12% beträgt.

23. Verwendung nach einem der Ansprüche 1 bis 12, **dadurch gekennzeichnet, dass** die Formulierung entweder als nicht-wässrige oder wässrige Formulierung eingesetzt wird.

24. Verwendung nach einem der Ansprüche 1 bis 12, **dadurch gekennzeichnet, dass** Azeton und Propylenglykol als Permeationsbeschleuniger verwendet werden.

25. Verwendung nach einem der Ansprüche 1 bis 24 zur Behandlung von Innenohrerkrankungen.

26. Verwendung nach einem der Ansprüche 1 bis 24 zur Behandlung des muskulären oder myognatischenTinnitus.

27. Verwendung nach einem der Ansprüche 1 bis 24 zur Behandlung der Morbus Ménière.

28. Verwendung nach einem der Ansprüche 1 bis 24 zur Behandlung von Sprachdiskriminationsstörungen, insbesondere solche im Zusammenhang mit Hörstörungen.

29. Verwendung nach einem der Ansprüche 1 bis 24 zur Behandlung des labyrinthären Schwindels.

## Revendications

1. Utilisation d'un composé de quinoxalin-2-one de la formule 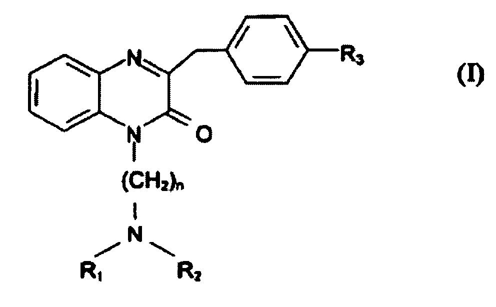 selon laquelle
R1 et R2, indépendamment l'un de l'autre, sont de l'hydrogène, du méthyle, de l'éthyle, du propyle ou du butyle ou R1 et R2 forment ensemble un composé cyclo-alkyle ;
R3 est un groupe méthoxy, éthoxy, hydroxy, de l'hydrogène, un alkyle en C1-C4 ou un halogène et n = 1, 2 ou 3,
ou un sel compatible sur le plan pharmaceutique du composé de quinoxalin-2-one sus-mentionné, ensemble avec un accélérateur de perméabilité ou un support par rapport au composé de quinoxalin-2-one-1-un, pour la production d'une formulation pharmaceutique sous forme d'un liquide ou de liposomes ou d'une nano-émulsion pour administration transtympanique.

2. Utilisation selon la revendication 1 selon laquelle R1 et R2 sont des groupes éthyles, n = 2 et R3 est un groupe méthoxy si bien que la molécule est 1-diéthyl-aminoéthyl-3-(p-méthoxybenzyl)-1,2-dihydro-quinoxalin-2-one ou un sel de celle-ci compatible sur le plan pharmaceutique.

3. Utilisation selon la revendication 1 selon laquelle R1 et R2 sont des groupes éthyles, n = 2 et R3 est un groupe hydroxy si bien que la molécule est 1-diéthyl-aminoéthyl-3-(p-hydroxybenzyl)-1,2-dihydro-quinoxalin-2-one ou un sel de celle-ci compatible sur le plan pharmaceutique.

4. Utilisation selon l'une quelconque des revendications 1 à 3 selon laquelle l'accélérateur de perméabilité contient au moins du sulfoxide diméthylique ou du propylène glycol.

5. Utilisation selon la revendication 4 selon laquelle la part en poids de sulfoxide diméthylique utilisée dans la formulation est entre 5 et 50 %.

6. Utilisation selon l'une des revendications 1 à 5 selon laquelle au moins un autre second accélérateur de perméabilité est contenu en combinaison avec le sulfoxide diméthylique.

7. Utilisation selon la revendication 6 selon laquelle le second accélérateur de perméabilité est un composé de glycol.

8. Utilisation selon la revendication 6 ou 7 selon laquelle le second accélérateur de perméabilité est l'éthylène glycol et/ou le propylène glycol.

9. Utilisation selon l'une quelconque des revendications 1 à 8 selon laquelle le rapport en poids de quinoxalin-2-one et de l'accélérateur de perméabilité est entre 1:2 et 1:500, de préférence entre 1:20 et 1:100.

10. Utilisation selon l'une quelconque des revendications 1 à 9 selon laquelle il est utilisé en plus un solvant qui est la glycérine et/ou l'eau.

11. Utilisation selon l'une quelconque des revendications 1 selon laquelle une nano-émulsion ou des liposomes qui contiennent ledit composé de quinoxalon-2-one selon la formule (I) sont utilisés comme accélérateur de perméation ou support.

12. Utilisation selon la revendication 11 selon laquelle la nanoémulsion ou les liposomes contiennent les composés suivants en plus dudit composé de quinoxalon-2-one :
- une molécule formatrice de membrane et
- un co-émulsifiant.

13. Utilisation selon la revendication 12 selon laquelle la molécule formatrice de membrane est une substance qui rend possible la formation d'un système bi-couche.

14. Utilisation selon la revendication 12 ou 13 selon laquelle la molécule formatrice de membrane est un phospholipide hydrogéné ou partiellement hydrogéné.

15. Utilisation selon la revendication 14 selon laquelle le phospholipide est la phosphatidylcholine, la phosphatidyléthanolamine, le phosphatidylglycérol, le phosphatidylinositol, la phosphatidylsérine et la sphingomyéline dans lequel la chaîne acyle peut être soit saturée, soit insaturée et peut avoir entre 12 et 22 atomes de C, de préférence entre 14 et 18 atomes de C.

16. Utilisation selon la revendication 14 ou 15 selon laquelle le phospholipide a la formule 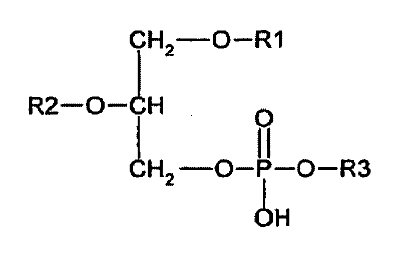 dans laquelle
R1 indique un acyle en C₁₀-C₂₀ ;
R2 indique l'hydrogène ou un acyle en C₁₀-C₂₀,
R3 indique l'hydrogène, le 2-triméthylamino-1-éthyle, le 2-amino-1-éthyle, un alkyle en C₁-C₅ non substitué ou un alkyle en C₁-C₅ substitué par un ou plusieurs groupes carboxyles, hydroxyles ou amino ; l'inositol ou le groupe glycérile ou de sels de ces composés est utilisé comme molécule formatrice de membrane.

17. Utilisation selon l'une des revendications 12 à 16 selon laquelle le composant formateur de membrane est utilisé dans une concentration d'environ 0,1 à 30 % en poids, exprimée en termes de poids total de composant formateur de membrane, d'émulsifiant et de substance active.

18. Utilisation selon la revendication 12 selon laquelle les co-émulsifiants suivants ou un mélange d'émulsifiant de deux ou de plusieurs co-émulsifiants dont la liste suit est utilisé : métal alcalin, sels d'ammonium et d'aminium d'acides gras, par exemple sels de lithium, sodium, potassium, ammonium, triéthylamine, éthanolamine, diéthanolamine ou triéthanolamine. Les sels de sodium, potassium ou d'ammonium (NR₁R₂R₃) sont préférés, R₁, R₂ et R₃, indépendamment l'un de l'autre indiquant l'hydrogène, un alkyle en C₁-C₄ ou un hydroxyalkyle en C₁-C₄.
Des sulfates alkyles tels que, par exemple, le dodécylsulfate de sodium.
Des sels d'acide biliaire, par exemple cholate de sodium, glycocholate de sodium et taurocholate de sodium ;
des esters d'acides gras partiel de sorbitane, par exemple monolaurate de sorbitane;
des esters de sucre, par exemple monolaurate de sucrose ;
des glycérides partiels d'acides gras, par exemple monoglycéride d'acide laurique ;
des esters de polyglycérol d'acides gras ;
des esters de propylène glycol d'acides gras ;
des esters d'acide lactique d'acides gras, par exemple le sodium stéaroyl-lactyl-2-lactate ;
des protéines, par exemple la caséine.

19. Utilisation selon la revendication 18 selon laquelle les émulsifiants du type polyoxyéthylène sont :
- des esters d'acides gras polyéthoxylatés de sorbitane, par exemple du polysorbate 80 ;
- des dérivés polyéthoxylatés de vitamine E, par exemple succinate 1000 de polyéthylène glycol vitamine E ;
- des glycérides partiels polyéthoxylatés d'acides gras, par exemple monostéarate de diéthylène glycol ;
- des hydrates de carbone polyéthoxylatés ;
- des polymères bloc d'oxyde d'éthylène et d'oxyde de propylène, par exemple du poloxamer 188.

20. Utilisation selon l'une quelconque des revendications 12 à 19 selon laquelle l'émulsifiant est présent dans la formulation à une concentration d'environ 1 à environ 50 % en poids, exprimée en termes de poids total du composant formateur de membrane, de l'émulsifiant et de la substance active.

21. Utilisation selon l'une quelconque des revendications 12 à 20 selon laquelle le composé de quinoxalin-2-one est présent dans la formulation à une concentration d'environ 0,1 à environ 70 % en poids, exprimée en termes de poids total des composants (composant formateur de membrane, émulsifiant et composé de quinoxalin-2-one).

22. Utilisation selon l'une quelconque des revendications 1 à 12 selon laquelle la formulation est liquide et la part en poids du composé de quinoxalin-2-one est entre 0,5 % et 12 %.

23. Utilisation selon l'une quelconque des revendications 1 à 12 selon laquelle la formulation est utilisée soit comme formulation non aqueuse, soit comme formulation aqueuse.

24. Utilisation selon l'une quelconque des revendications 1 à 12 selon laquelle l'acétone et le propylène glycol sont utilisés comme accélérateurs de perméation.

25. Utilisation selon l'une quelconque des revendications 1 à 24 selon laquelle il est utilisé pour le traitement des maladies de l'oreille interne.

26. Utilisation selon l'une quelconque des revendications 1 à 24 selon laquelle il est utilisé pour le traitement de l'acouphène musculaire ou myognathique.

27. Utilisation selon l'une quelconque des revendications 1 à 24 selon laquelle il est utilisé pour le traitement de la maladie de Ménière.

28. Utilisation selon l'une quelconque des revendications 1 à 24 selon laquelle il est utilisé pour le traitement de déficience discriminatoire du langage, en particulier combinée à une déficience auditive.

29. Utilisation selon l'une quelconque des revendications 1 à 24 selon laquelle il est utilisé pour le traitement de vertiges labyrinthiques.
